# EUROPEAN PATENT APPLICATION

(11) **EP 1 875 925 A1**
(43) Date of publication of application: **09.01.2008**
(21) Application number: 06732092.9
(22) Date of filing: 19.04.2006
(51) Int. Cl.: A61K 45/00, A61K 31/4439, A61P 1/04, A61P 1/12, C07D 401/12

(54) **PREVENTIVE OR REMEDY FOR BOWEL DISEASE**

(30) Priority: 22.04.2005 JP 2005124712; 22.04.2005 JP 2005124713
(71) Applicant: Astellas Pharma Inc., Tokyo 103-8411 (JP)
(72) Inventor: ISHIKAWA, Jun, c/o Astellas Pharma Inc., Tokyo 103-8411 (JP); TAKEZAWA, Ryuichi, c/o Astellas Pharma Inc., Tokyo 103-8411 (JP); MASUNAGA, Youhei, Shizuoka 4338122 (JP)
(74) Representative: HOFFMANN EITLE
(86) International application number: PCT/JP2006/308185
(87) International publication number: WO 2006/115140

(57) **Abstract**

[Problem]

To provide a preventive or a remedy for various diarrheas caused by inflammatory colitis, irritable bowel syndrome and other factors.

[Means for Solution]

It was found for the first time that CRAC/SOC channels play a physiologically important role in water secretion or absorption in gastrointestinal epithelia cells and a CRAC/SOC channel inhibitor is useful for preventing and treating diarrhea caused by various factors, and a preventive and a remedy for diarrhea comprising a compound inhibiting the calcium-release-activated calcium channels as an active ingredient, particular a remedy for diarrheal symptoms associated with inflammatory bowel diseases and a remedy for irritable bowel syndrome are provided.

## Description

### TECHNICAL FILED

The present invention relates to a preventive or a remedy for bowel diseases, which comprises, as an active ingredient, a compound inhibiting a calcium release-activated calcium channel.

### BACKGROUND ART

Well known diarrheas include infectious diarrhea induced by infection with viruses, bacteria or parasites (cf. Non-Patent Reference 1 and Non-Patent Reference 2); and non-infectious diarrhea caused by stress (cf. Non-Patent Reference 3 and Non-Patent Reference 4), antibiotics (cf. Non-Patent Reference 5), lactose intolerance (cf. Non-Patent Reference 6) allergic reaction (cf. Non-Patent Reference 7), etc. Recently, diarrhea associated with inflammatory bowel diseases and diarrhea by irritable bowel syndrome have become problems.
Inflammatory bowel diseases are bowel disorders of which the cardinal sign is a diarrheal symptom with abdominal pain. Inflammatory bowel diseases are principally grouped into Crohn's disease and ulcerative colitis; and Crohn's disease is characterized by penetrating deep inflammation, while ulcerative colitis is by inflammation limited in mucosa and submucosa. Crohn's disease is an inflammatory disease in which the lesion distribution is non-continuous and a normal site exists between the lesion sites; and in this, since the inflammation is deep, fistulae are formed. In the lesion site in Crohn's disease, there is seen infiltration of T-lymphocytes, neutrophils, monocytes and activated macrophages, and it is considered that cytokines, chemokines and active oxygen produced by the infiltrating inflammatory cells may injure gastrointestinal epithelial tissues (cf. Non-Patent Reference 8). In ulcerative colitis, uniform inflammations are seen almost entirely in the colonic mucosa, an in the lesion site, there exists lacunar abscess. In ulcerative colitis, there is seen infiltration of T-lymphocytes, antibody-producing cells, neutrophils, eosinophils, monocytes and macrophages, and it is considered that cytokines, chemokine, active oxygen and antibody production from the infiltrating cells may injure colonic mucosal tissues (cf. Non-Patent Reference 9 and Non-Patent Reference 10).

Irritable bowel syndrome is a bowel disorders of which the cardinal sign is a diarrheal symptom with abdominal pain, like that of inflammatory bowel diseases; and at present, it is considered that this may be a disease essentially caused by the abnormality of gastrointestinal plexuses via a sensory neurotransmitter such as typically serotonin. Concretely, hypersecretion of a sensory neurotransmitter such as serotonin in gastrointestinal plexuses may result in homeostatic gastrointestinal motility failure and excessive secretion from gastrointestinal epithelial tissues, thereby causing diarrheal symptoms with abdominal pain (cf. Non-Patent References 11 to 13).

On the other hand, calcium release-activated calcium channels (Ca²⁺ release-activated Ca²⁺ (CRAC) channels) are, as another term, referred to as calcium store-operated calcium channels (store-operated Ca²⁺ (SOC) channels) or calcium store depletion-activated calcium channels (depletion-activated Ca²⁺ channels), and these are channels that regulate the intracellular calcium concentration as a intracellular transmitter, and participates in capacitative calcium entry (capacitative Ca²⁺ entry) or intracellular calcium oscillation (intracellular Ca²⁺ oscillation) during cell activation. Regarding the CRAC or SOC channels (hereinafter, CRAC/SOC channels or CRAC channels), the molecule itself is not as yet identified, but up to the present from the studies based on a CRAC/SOC channel-specific calcium entry current, CRAC current (CRAC current (I_{CRAC})) as the index thereof, it is reported that the channel is distributed typically in many inflammatory cells and in some tissues such as endothelial cells or epithelial cells (cf. Non-Patent Reference 14). In addition, the CRAC/SOC channels play an important role in a sustained calcium entry in cell activation. In particular, it is known that, in immunocytes and inflammatory cells, continuous calcium entry via a CRAC/SOC channels regulate the activation of a cytokine production-related transcription factor, a nuclear factor of activated T cells (NF-AT) (cf. Non-Patent References 15 and 16) or a nuclear factor-κB (NFκB) (cf. Non-Patent Reference 17); and it is reported that, since its inhibitor remarkably inhibits the activation of immunocytes and inflammatory cells and exhibits a broad-range antiinflammatory effect, the inhibitor is effective for contact dermatitis, asthma and rheumatoid arthritis in diseased animal models (cf. Patent Reference 1, Patent Reference 2, Non-Patent Reference 18).

It is reported that the CRAC/SOC channels also express in epithelial cells such as typically those in intestinal tracts (cf. Non-Patent References 19 to 22, and Non-Patent Reference 14). In addition, it is reported that a calcium ATPase (Ca²⁺-ATPase) inhibitor thapsigargin used for induction of CRAC/SOC channel activation causes chloride secretion or short circuit current (I_{sc}) increase accompanied by the intracellular calcium concentration increase in epithelial cells (cf. Non-Patent Reference 23 and Non-Patent Reference 24). However, there is known no report as to whether or not a CRAC/SOC channel inhibitor may have a remedial or preventive effect for diarrhea.
4,6-Dimethyl-4'-[3,5-bis(trifluoromethyl)-1H-pyrazol-l-yl]nicotinanilide (hereinafter abbreviated as "compound A") or its salt is a compound included by the CRAC channel-inhibiting pyrazole derivative disclosed by Patent Reference 1. Patent Reference 2 discloses a preferred crystal of the compound A, saying that the compound A is useful as a preventive or a remedy for CRAC channel or IL-2-related allergic, inflammatory or autoimmune diseases and concretely showing various diseases such as bronchial asthma as well as inflammatory bowel diseases including Crohn's disease as the allergic, inflammatory or autoimmune diseases. However, it discloses no pharmacological test results for concretely demonstrating the effect. On the other hand, Patent Reference 1 that includes the compound A discloses *in vitro* test results of CRAC channel inhibitory effect and IL-2 production inhibitory effect and also various test results with an antigen-induced tracheobronchial eosinophilic infiltration model that is a typical diseased model of bronchial asthma, a mouse TNCB-induced contact hypersensitivity model, a mouse ConA-induced hepatitis model, and a mouse collagen-induced arthritis model; however, it does not disclose a pharmacological test and its results relating to inflammatory bowel diseases.

Non-Patent Reference 1: Gut 53, 296-305 (2004)
Non-Patent Reference 2: New Eng. J. Med. 350, 38-47 (2004)
Non-Patent Reference 3: Pharmacol. Toxicol. 90, 109-120 (2002)
Non-Patent Reference 4: Dig Dis 19, 201-211 (2001)
Non-Patent Reference 5: New Eng. J. Med. 346, 334-339 (2002)
Non-Patent Reference 6: Gastroenterol. 76, 365-74 (1979)
Non-Patent Reference 7: Minerva Med. 93, 403-412 (2002)
Non-Patent Reference 8: Lancet 359, 62-69 (2002)
Non-Patent Reference 9: N Eng. J Med. 347, 417-429 (2002)
Non-Patent Reference 10: Curr. Opin. Gastroenterol. 20, 345-50 (2004)
Non-Patent Reference 11: Br. J. Pharmacol. 141, 1285-1293 (2004)
Non-Patent Reference 12: New Eng. J. Med. 349, 2136-2146 (2003)
Non-Patent Reference 13: Lancet 360, 555-564 (2002)
Non-Patent Reference 14: Physiol. Rev. 77, 901-30 (1997)
Non-Patent Reference 15: J. Cell. Biol. 131, 655-67 (1995)
Non-Patent Reference 16: J. Immunol. 159, 1628-38 (1997)
Non-Patent Reference 17: J. Immunol., 157, 5277-5283 (1996)
Non-Patent Reference 18: J. Immunol. 170, 4441-9 (2003)
Non-Patent Reference 19: J. Biol. Chem. 270, 29169-29175 (1995)
Non-Patent Reference 20: Am. J. Physiol. 277, L1089-L1095 (1999)
Non-Patent Reference 21: Cell. Calcium 33, 357-373 (2003)
Non-Patent Reference 22: J. Gen. Physiol. 122, 207-223 (2003)
Non-Patent Reference 23: J. Clin. Invest. 98, 2066-2075 (1996)
Non-Patent Reference 24: Am. J. Physiol. 275, C484-C495 (1998)
Patent Reference 1: WO 99/19303
Patent Reference 2: WO 04/020433

### DISCLOSURE OF THE INVENTION

### PROBLEMS THAT THE INVENTION IS TO SOLVE

Prevention and treatment of inflammatory bowel diseases and diarrhea, especially diarrhea with abdominal pain is important for patients in the meaning of improving the quality of life (QOL) thereof. Still now, it is desired to develop effective remedies.

### MEANS FOR SOLVING THE PROBLEMS

The present inventors have assiduously studied the pharmacological effect of CRAC/SOC channel inhibitors and, as a result, have found that a CRAC/SOC channels play an physiologically important role in water secretion or absorption in gastrointestinal epithelial cells, that a CRAC/SOC channel inhibitor exhibits a good therapeutical effect in various diarrheal animal models and is clinically useful for prevention and treatment of diarrhea to be caused by various factors, and further that, since the CRAC/SOC channel inhibitor has both an antiinflammatory effect and a directly improving effect for diarrheal symptoms, it is useful as a preventive or a remedy for inflammatory bowel diseases, especially inflammatory bowel diseases accompanied by diarrheal symptoms, and thus have herein completed the present invention.
Specifically, the present invention relates to a preventive or a remedy for diarrhea that comprises, as the active ingredient thereof, CRAC/SOC channel-inhibiting compounds, especially to a preventive or a remedy for diarrheal symptoms associated with inflammatory bowel diseases and for diarrhea in irritable bowel syndrome, as well as to a preventive or a remedy for inflammatory bowel diseases.
The CRAC-SOC channel-inhibiting compound is preferably a pyrazole derivative of the following general formula (I) or a pharmaceutically acceptable salt thereof, especially preferably 4,6-dimethyl-4'-[3,5-bis(trifluoromethyl)-1H-pyrazol-1-yl]nicotinanilide or a salt thereof. 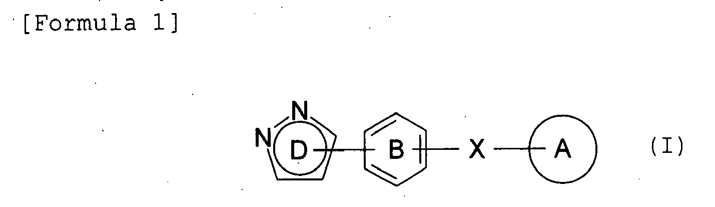 (wherein the symbols in the formula have the following meanings:
D: a 1H-pyrazol-5-yl or 1H-pyrazol-1-yl group substituted with one trifluoromethyl group and optionally having one or two substituents selected from the group consisting of lower alkyl, halogeno-lower alkyl, -CO₂H and -CO₂-lower alkyl;
X: -NHCO- or -CONH-;
A: a phenyl group optionally substituted with halogen atom(s), or a monocyclic heteroaryl group selected from thiazolyl, thiadiazolyl, thienyl and pyridyl groups, which may optionally be substituted with lower alkyl group(s);
B: a phenylene group; and the same shall apply hereinunder).

### EFFECT OF THE INVENTION

The preventive or a remedy of the present invention has a good antiinflammatory effect and in addition, it may directly act on the CRAC/SOC channels in gastrointestinal epithelial cells to inhibit water section, and is therefore useful as a preventive or a remedy for inflammatory bowel diseases and/or diarrhea, especially for prevention and treatment of inflammatory bowel diseases that are bowel disorders expressing, as the cardinal sign thereof, a diarrheal symptom with abdominal pain. The diarrhea includes infectious diarrhea to be induced by infection with viruses, bacteria or parasites; non-infectious diarrhea to be caused by stress, antibiotics, lactose intolerance or allergic reaction; diarrheal symptoms associated with inflammatory bowel diseases as well as diarrhea in irritable bowel syndrome.

### BRIEF DESCRIPTION OF THE DRAWINGS

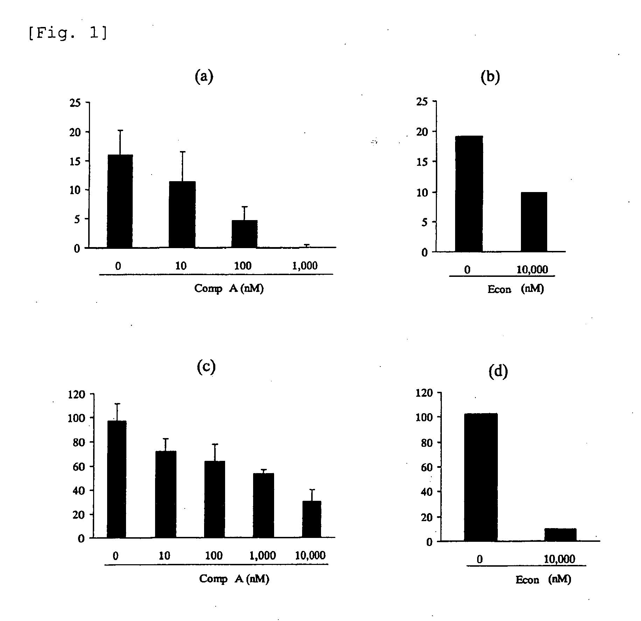

[Fig. 1] Fig. 1 includes graphs showing the effect of the compound A (Comp A) and econazole (Econ) in Example 2 on chloride-dependent short circuit current (I_{sc}) (uA/cm²) increase. (a) shows the effect of Comp A on thapsigargin (1 µM) -induced Isc increase; (b) shows the effect of Econ on thapsigargin (1 µM) - induced I_{sc} increase; (c) shows the effect of Comp A on forskolin (10 µM)-induced Isc increase; and (d) shows the effect of Econ on forskolin (10 µM)-induced I_{sc} increase. The vertical axis of the graphs indicates a chloride-dependent short circuit current (Isc) (µA/cm²).

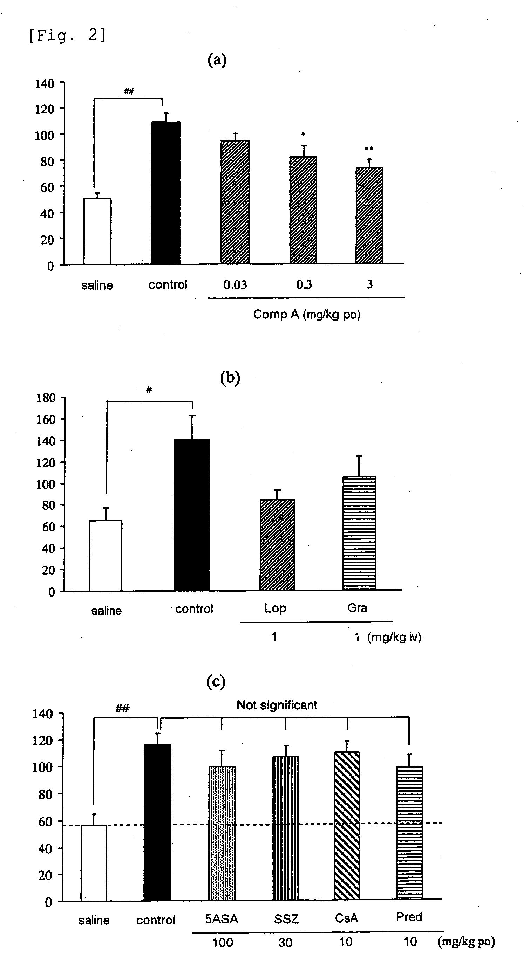

[Fig. 2] Fig. 2 includes graphs showing the inhibitory effects of test compounds on mouse cholera toxin-induced intestinal hypersecretion models in Example 3. (a) shows the results of compound A (Comp A) administration groups; (b) shows the results of loperamide (Lop) or granisetron (Gra) administration groups; (c) shows the results of antiinflammatory/immunomodulatory agent, 5-aminosalicylic acid (5ASA), sulfasalazine (SSZ), cyclosporine A (CsA) or prednisolone (Pred) administration groups. The vertical axis of the graphs indicates an ileal loop weight per unit (mg/cm). # and ## each show a significant difference (p < 0.05 and p < 0.01) compared with a 0.9% physiological saline (saline)-treated group; and * and ** each show a significant difference (p < 0.05 and p < 0.01) compared with a control (Control) group.

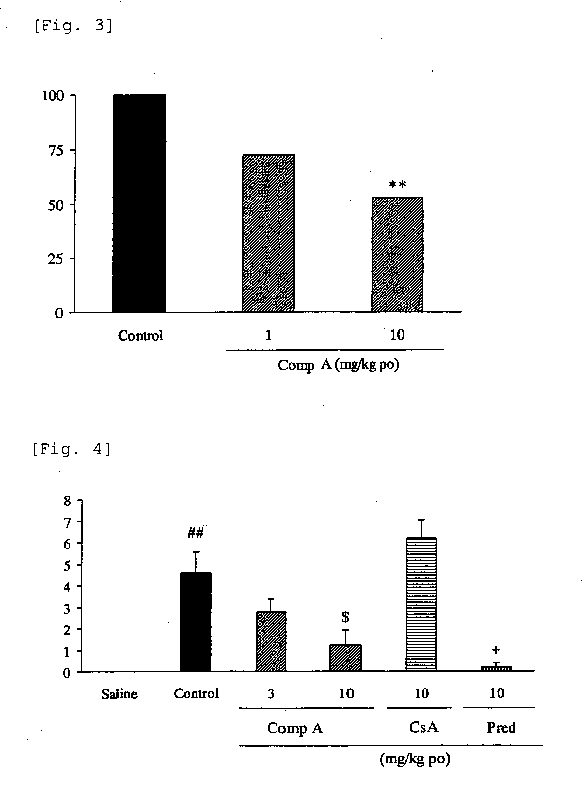

[Fig. 3] Fig. 3 shows the effect of the compound A (Comp A) administration groups on rat stress-induced diarrheal models in Example 4. The vertical axis indicates an incidence rate (Control %) based on a control group of 100%. ** shows a significant difference (p < 0.01) compared with the control (Control) group.

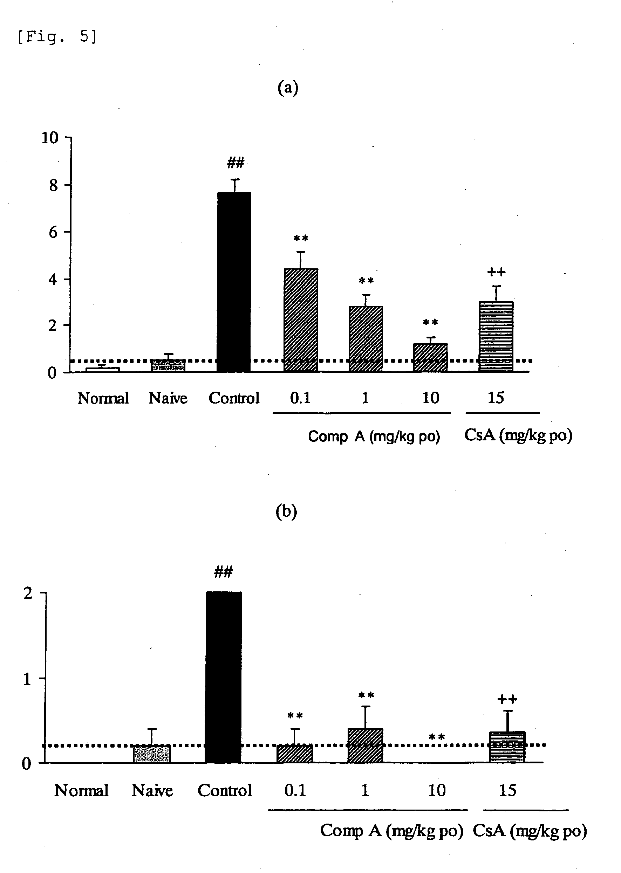

[Fig. 4] Fig. 4 is a graph showing the effects of compound A (Comp A) administration groups, cyclosporine A (CsA) administration groups and prednisolone (Pred) administration groups on mouse antigen-induced diarrheal models in Example 5. The vertical axis shows the sum total of the diarrheal reaction score on days 27 to 38 with diarrheal onset. ## shows a significant difference (p < 0.01) compared with a 0.9% physiological saline (saline)-treated group; and + and $ each show a significant difference (p < 0.05 and p < 0.01) compared with a control (Control) group.
[Fig. 5] Fig. 5 includes graphs showing the effects of compound A (Comp A) administration groups and cyclosporine A (CsA) administration groups on rat oxazolone-induced acute colitis models in Example 6. The vertical axis in (a) indicates a disease activity index score; and that in (b) indicates a fecal condition score; and ** and ++ each show a significant difference (both p < 0.01) compared with a control (Control) group.

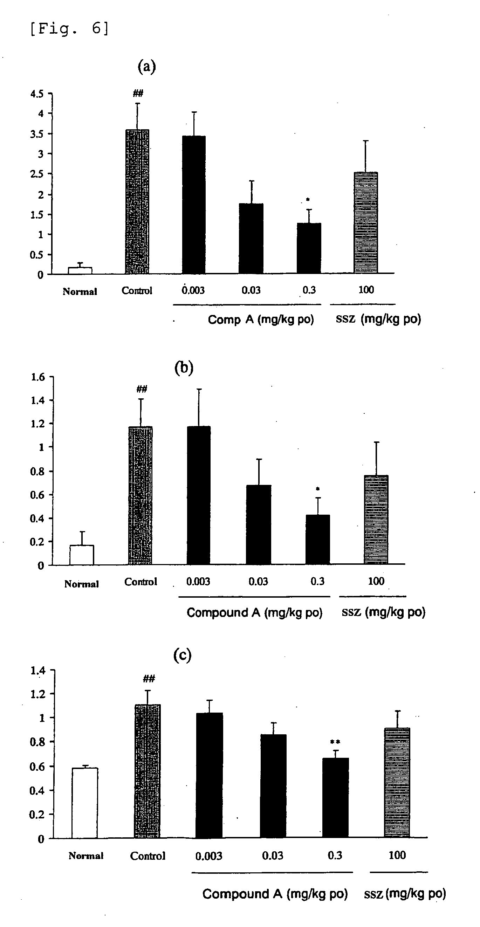

[Fig. 6] Fig. 6 includes graphs showing the effects of compound A (Comp A) administration groups and sulfasalazine (SSZ) administration groups on rat TNBS-induced chronic colitis models in Example 7. The vertical axis in (a) indicates a disease activity index score; that in (b) indicates a fecal condition score; and that in (c) indicates an MPO activity (mU/cm tissue). ++ shows a significant difference (p < 0.01) compared with a non-treated (naive) group; * and ** each show a significant difference (p < 0.05 and p < 0.01) compared with a control (Control) group.

### BEST MODE FOR CARRYING OUT THE INVENTION

The CRAC/SOC channel inhibitor is preferably econazole (Br. J. Pharmacol. 119: 647-54 1996), SK&F-96365 (Br. J. Pharmacol. 113: 861-8 1994), and the pyrazole derivative disclosed in the above-mentioned Patent Reference 1. These CRAC/SOC channel inhibitors are commercially sold, or are readily available according to methods described in literature. For example, the compounds of formula (I), which are preferred CRAC/SOC channel inhibitors of the present invention, as well as 4,6-dimethyl-4'-[3,5-bis(trifluoromethyl)-1H-pyrazol-1-yl]nicotinanilide (hereinafter, compound A) are readily available according to the method described in the above-mentioned Patent Reference 1 or 2. Further, the CRAC/SOC channel inhibitor of the present invention includes a combination of two or more of the above-mentioned, CRAC/SOC channel-inhibitory activity-having compounds.
In the compounds of formula (I), "lower alkyl" is a linear or branched alkyl having from 1 to 6 carbon atoms, and is preferably methyl, ethyl, propyl. "Halogen atom" includes I, Br, F and Cl. "Halogeno-lower alkyl" is a lower alkyl substituted with at least one halogen atoms, and is especially preferably fluoromethyl, difluoromethyl, trifluoromethyl. "Phenyl group optionally substituted with halogen atom(s)" for A means a phenyl group which is unsubstituted or substituted with from 1 to 5 halogen atoms; and "monocyclic heteroaryl group selected from thiazolyl, thiadiazolyl, thienyl and pyridyl groups and optionally substituted with a lower alkyl group" means a thiazolyl, thiadiazolyl, thienyl or pyridyl group which is unsubstituted or substituted with from 1 to 3 lower alkyl groups (in case where the group is substituted with plural substituents, they may be the same or different). Preferably, D is a 1H-pyrazol-1-yl-group substituted with two trifluoromethyl groups, X is -NHCO-, A is a pyridyl group which is unsubstituted or substituted with one or two methyl groups, and B is 1,4-phenylene.

The pyrazole derivatives of formula (I) and the compound A, which are preferred CRAC/SOC channel inhibitors of the present invention, may form acid-addition salts or salts with a base; and so far as such salts are pharmaceutically-acceptable salts, they are included by the CRAC/SOC channel inhibitor of the present invention. Concretely, they include acid-addition salts with an inorganic acid such as hydrochloric acid, hydrobromic acid, hydroiodic acid, sulfuric acid, nitric acid, phosphoric acid, or an organic acid such as formic acid, acetic acid, propionic acid, oxalic acid, malonic acid, succinic acid, fumaric acid, maleic acid, lactic acid, malic acid, tartaric acid, citric acid, methanesulfonic acid, ethanesulfonic acid, aspartic acid, glutamic acid; salts with an inorganic base such as sodium, potassium, magnesium, calcium, aluminium, or an organic base such as methylamine, ethylamine, ethanolamine, lysine, ornithine; and ammonium salts. These compounds may be various types of their hydrates or solvates, and include their polymorphic substances. Pharmaceutically acceptable prodrugs of these compounds are also included by the CRAC/SOC channel inhibitor of the present invention. Pharmaceutically-acceptable prodrugs are compounds having a group capable of being converted into NH₂, OH, CO₂H or the like in the present invention through solvolysis or under a physiological condition. The group to form prodrugs includes those described in Prog. Med., 5, 2157-2161 (1985); and Pharmaceutical Research and Development (Hirokawa Shoten, 1990), Vol. 7, Drug Design, 163-198.

Pharmaceutical compositions containing, as an active ingredient thereof, the CRAC/SOC channel inhibitor of the present invention may be prepared according to ordinary methods, using carriers, vehicles and other additives generally used in pharmaceutical formulation.
The administration may be in any route of oral administration with tablets, pills, capsules, granules, powders or liquids, or parenteral administration with injections such as intravenous injections or intramuscular injections, or suppositories, transdermal agents, nasal agents, inhalants or intravesical injections. The dose may be suitably determined for individuals, in consideration of the condition, the age and the sex of the subject for administration. In oral administration, in general, the dose may be from 0.001 mg/kg adult/day to 100 mg/kg adult/day, preferably from 0.01 mg/kg adult/day to 10 mg/kg adult/day. This may be administered all at a time, or as divided for administration in 2 to 4 times. For intravenous administration depending on the condition, in general, the dose may be from 0.0001 mg/kg adult/day to 10 mg/kg adult/day, preferably from 0.001 mg/kg adult/day to 1 mg/kg adult/day. This may be administered at a time in a day, or as divided for administration in plural times in a day. For inhalation, in general, the dose may be from 0.0001 mg/kg adult/day to 1 mg/kg adult/day. This may be administered at a time in a day, or as divided for administration in plural times in a day.

Tablets, powders and granules may be used as the solid composition for oral administration. The solid composition may contain one or more active substances, as mixed with at least one inert vehicle, such as lactose, mannitol, glucose, hydroxypropyl cellulose, microcrystalline cellulose, starch, polyvinyl pyrrolidone, magnesium metasilicate aluminate. According to an ordinary manner, the composition may contain inert additives, for example, lubricant such as magnesium silicate, disintegrator such as carboxymethyl starch-sodium, solubilizer or dissolution promoter. The pills or tablets may be coated with sugar or with gastric-coating or enteric-coating film.
The liquid composition for oral administration includes pharmaceutically-acceptable emulsion, solution, suspension, syrup and elixir, and contains an ordinary inert solvent such as pure water, ethanol. The composition may contain any other additive than such an inert solvent, for example, auxiliary agent such as solubilizer, wetting agent, suspending agent, as well as sweetener, flavoring, fragrance, and preservative.

The injection for parenteral administration includes germ-free water-base or waterless solution, suspension and emulsion. The water-base solvent includes, for example, distilled water for injection and physiological saline water. The waterless solvent includes, for example, propylene glycol, polyethylene glycol, vegetable oil such as olive oil, alcohols such as ethanol, Polysorbate 80 (name by Pharmacopeia of Japan). The composition may further contain isotonizer, preservative, wetting agent, emulsifier, dispersant, stabilizer, solubilizer, dissolution promoter. These may be sterilized through filtration through a bacteria-trapping filter, or by addition of germicide, or through irradiation with light. As the case may be, a germ-free solid composition may be prepared, and it may be dissolved or suspended in germ-free water or germ-free solvent for injection to give the intended liquid composition before use.

The intramucosal composition such as inhalant and nasal agent may be solid, liquid or semi-solid, and it may be produced according to a known method. For example, vehicles such as lactose and starch, and further pH-controlling agent, preservative, surfactant, lubricant, stabilizer and thickener may be suitably added. For its administration, usable is any suitable device for inhalation or insufflation. For example, using a known device or spray such as a metered dose inhalation device, the compound may be administered singly, or as a formulated mixture powder thereof or as a solution or suspension thereof combined with a pharmaceutically-acceptable carrier. The dry powder inhalator may be for single-dose administration or multi-dose administration, for which dry powder or powder-containing capsules may be used. As the case may be, it may be in the form of a pressure aerosol spray using a suitable propellant, for example, a favorable vapor such as chlorofluoroalkane, hydrofluoroalkane or carbon dioxide.

### EXAMPLES

The present invention is described concretely with reference to the following Examples, which, however, do not restrict the scope of the present invention.

### Example 1 - CRAC/SOC channel inhibitory effect:

100 µL of a suspension of Jurkat T cells (6 × 10⁶ cells/mL) loaded with a calcium fluorescent indicator dye, fura-2 (1 µM) was put into the wells of a 96-well microplate. Thapsigargin-stimulated intracellular calcium concentration increase was caused by adding a test chemical and thapsigargin (final concentration, 1 µM) to each well. 30 minutes after their addition, the fluorescent intensity at excitation wavelength 340 nm/500 nm and excitation wavelength 380 nm/500 nm is measured, and the ratio of the thus-obtained two fluorescent intensity data indicates the concentration increase. From the intracellular calcium concentration in the presence of a test compound at a varying concentration thus obtained, and the intracellular calcium concentration in the control group with a solvent alone thus obtained, the calcium entry inhibitory (CRAC/SOC channel inhibitory) percentage by the test compound at a varying concentration was determined, and the concentration for 50% CRAC/SOC channel inhibition (IC₅₀) was computed (the above-mentioned Patent Reference 1 is referred to for its details).
Econazole and the compound A dose-dependently inhibited the thapsigargin-induced a sustained calcium entry, and their inhibitory activity was 14 and 0.3 µM, respectively.

### Example 2 - Inhibitory effect on secretion from human intestinal epithelial cells:

It is known that water secretion from epithelial cells is correlated with chloride ion secretion, and the effect to water secretion from epithelial cells may be evaluated through chloride-dependent current measurement.
T84 cells, a human colon epithelial cell-derived cell line, were purchased from American Type Culture Collection (ATCC, USA). T84 cells (3 × 10⁵ cells/well) were cultured on a polyethylenic mesh insert (Transwell® by Corning, USA)) on a 6-well microplate for 10 to 12 days. As the culture medium, 5% bovine calf serum-containing DMEM/F-12 (Gibco, USA) was used. The cultured T84 cells were set in the sample chamber of an Ussing short-circuit current measuring system, and the chloride-dependent short circuit current (I_{sc}) was measured. I_{sc} was induced by adding thapsigargin (1 µM) or forskolin (10 µM) to the T84 cell monolayer specimen at its top on the membrane side thereof. The I_{sc} increase was completely inhibited by a non-selective chloride channel inhibitor, diphenylalanine-2-carboxylic acid (diphenylamine-2-carboxylic acid (DPC), 1 mM). In each test, the data obtained by DPC addition were taken as chloride-dependent I_{sc} base line data, and the effect of each test compound was revised.
The results are shown in Fig. 1. The CRAC/SOC channel inhibitors, econazole (Econ) and the compound A (Comp A) totally differ in their skeletons, but the two both inhibited the thapsigargin-induced continuous I_{sc} increase. In this, they did not affect on the transient I_{sc} increase seen before the sustained I_{sc} increase. In addition, the two compounds dose-dependently inhibited forskolin-induced I_{sc} increase.
The above results show that the compound A has an inhibitory effect on water secretion from gastrointestinal epithelial cells.

### Example 3 - Inhibitory effect on mouse cholera toxin-induced intestinal hypersecretion models:

Balb/c female mice were used in this test. Under anesthesia by ketamine (50 mg/kg) and xylazine (0.3 mg/kg) intraperitoneal administration thereto, the ileum-cecum was taken out from each animal through celiotomy. The ileum on the side of the cecum was ligated at two sites to form a loop having a length of about 3 cm, and 0.1 mL of cholera toxin (1 mg/mL) was injected into the loop to cause intestinal hypersecretion. In place of the cholera toxin, 0.9% physiological saline was injected in the same manner to the animals of a physiological saline (saline) group. The ileum loop was restored to the abdomen, and the abdomen was closed using a surgical stapler. Six hours after the cholera toxin treatment, the animals were sacrificed by dimethyl ether overanesthesia, and the ileum loop was taken out from the abdomen. The loop length and the loop weight were measured, and the secretion amount in the loop was calculated from the weight increase per the unit length. The CRAC/SOC channel inhibitor, compound A (Comp A), was orally administered (po) 24 hours and 1 hour before the cholera toxin treatment. To those of the control (control) group, a solvent, 0.5% methyl cellulose solution, was administered in the same manner. As comparative compounds, the following were tested in the same manner: Remedies for irritable bowel syndrome, serotonin antagonist (granisetron, Gra; 1 mg/kg intravenous injection (iv)) and opioid agonist (loperamide, Lop; 1 mg/kg iv); and antiinflammatory agents and immunomodulators used as remedies for inflammatory bowel diseases, 5-aminosalicylic acid (5ASA, 100 mg/kg po), sulfasalazine (SSZ, 30 mg/kg po), cyclosporine A (CsA, 10 mg/kg po) and prednisolone (Pred, 10 mg/kg po).
The results are shown in Fig. 2. The cholera toxin-treated control group showed a significant increase in the loop water content as compared with the saline group, indicating promoted intestinal hypersecretion. In the CRAC/SOC channel inhibitor, compound A-administered (Comp A) group, the cholera toxin-induced intestinal hypersecretion was dose-dependently inhibited; and the effect was significant in oral administration of 0.3 mg/kg or more. In the comparative compound, serotonin antagonist-administered (Gra) group and the opioid agonist-administered (Lop) group, the compound was also effective for the models. On the other hand, the already-existing antiinflammatory agents and immunomodulators all did not affect on the intestinal hypersecretion. From these, it may be presumed that the intestinal hypersecretion-inhibitory effect of the CRAC/SOC channel inhibitor of the present invention would not be expressed by the already-known antiinflammatory or immunomodulatory effect and mechanism but may be expressed via the water secretion inhibitory effect directly to gastrointestinal epithelial cells.

### Example 4 - Effect on rat stress-induced diarrheal models:

Wister male rats were used in this test. The animals were restrained in individual restrictive cages for 4 hours and given stress thereby to have diarrhea. Regarding the diarrheal condition thereof, the animals having defecated loose or more diarrheal stool after restrained for 4 hours were considered as those having diarrhea. 24 hours and 1 hour before the restraint, 1.0 or 10 mg/kg of the compound A (Comp A) was orally administered. Each group consisted of 19 to 20 animals. The statistical pharmaceutical potency analysis of the test compound was attained as a significant chi-square test, and its multiplicity was corrected by Bonferroni multiple comparison.
The results are shown in Fig. 3. The compound A (Comp A) dose-dependently inhibited the incidence rate of restrictive stress-induced diarrhea, and the 10 mg/kg oral administration group showed significant inhibition.

### Example 5 - Effect on mouse antigen-induced diarrheal models:

Balb/c female mice were used in this test. The animals were immunized through intraperitoneal administration of 50 µg of ovalbumin (OVA) and 1 mg of aluminium gel (alum), and after 2 weeks from the first immunization, they were again immunized under the same condition. After 2 weeks from the second immunization (Day 27), OVA (10 mg) was orally administered to the animals every other day for 10 days, whereby the animals were made to have diarrhea. The diarrheal condition was checked 1 hour after the OVA administration. Test compounds, Compound A (Comp A) 3 or 10 mg/kg, cyclosporine A (CsA) 10 mg/kg and prednisolone (Pred) 10 mg/kg were orally administered 1 hour before the OVA administration.
The results are shown in Fig. 4. The compound A (Comp A)-administered group dose-dependently inhibited the incidence rate of antigen-induced diarrhea. The Pred-administered group almost completely inhibited diarrhea. On the other hand, the CsA-administered group worsened the diarrheal symptom.
The above results of Examples 3 to 5 indicate that the CRAC/SOC channel inhibitor directly inhibits the water secretion from gastrointestinal epithelial cells in living bodies, and accordingly, these have confirmed for the first time the inhibitor could be a clinically good preventive and remedy for diarrhea.

### Example 6 - Effect on rat oxazolone-induced acute colitis models:

Dark agouti (DA) female rats were used in this test. After the hair was shaven on the abdominal region thereof, the rat was sensitized by applying 0.3 mL of 4% oxazolone onto the abdominal skin. In the non-sensitized (normal) group, 5% acetone + 95% ethanol was applied in place of oxazolone. One week after the sensitization with oxazolone, under anesthesia with ketamine (50 mg/kg) and xylazine (0.3 mg/kg) intraperitoneal administration, 0.2 mL of 3% oxazolone was injected into the colon at a position of about 8 cm from the anus using a polyethylenic tube,, thereby causing colitis. In the normal group and the non-treated (Naive) group, 0.5% methyl cellulose + peanut oil was injected in place of oxazolone. Twenty-four hours after colitis onset, the animals were sacrificed by dimethyl ether overanesthesia, and the anus-distal colon 6.5 cm was taken out. The stool condition and the ulcer symptom were evaluated by clinical indices and were scored. After its weight was measured, the colon tissue was frozen and stored, and analyzed for MPO activity measurement. Twenty-four hours before the treatment with oxazolone and 1 hour after it, a test compound was orally administered. In the control (Control) group, a solvent, 0.5% methyl cellulose solution was administered in the same manner. As a comparative compound, cyclosporine A (CsA), 15 mg/kg was orally administered in the same manner.
The results are shown in Fig. 5. In this test, the Control group that had colitis induced by oxazolone showed significant facilitation of diarrheal symptom, and showed intestinal tissue weight increase and exacerbation of inflammatory symptom indicated by MPO activity. On the other hand, the group with oral administration of the compound A (Comp A) 0.1 mg/kg, 1.0 mg/kg, or 10 mg/kg, a significant improvement effect on the disease activity index was confirmed, and diarrheal symptom relief, intestinal tissue weight increase inhibition and MPO activity promotion inhibition were observed. These results confirm that the CRAC/SOC channel inhibitor, compound A (Comp A) is effective for relieving both diarrheal symptom and inflammatory symptom. On the other hand, a typical immunomodulator, CsA also showed a relieving effect for the present models, but the effect was 1/50 times that of the compound A (Comp A). At least 0.1 mg/kg oral administration of the compound A (Comp A) almost completely inhibited the diarrheal symptom, and the compound exhibited its diarrhea-inhibiting effect even at a low dose of about 1/100 times that for its colitis symptom relieving effect.

### Example 7 - Effect on rat TNBS-induced chronic colitis models:

Wistar female rats were used in this test. Under anesthesia through intraperitoneal administration of ketamine (50 mg/kg) and xylazine (0.3 mg/kg) thereto, the animals were caused colitis by injection of 0.25 mL of trinitrobenzene-sulfonic acid (2,4,6-trinitrobenzene sulphonic acid (TNBS), 30 mg/0.25 mL 50% EtOH) into the colon at about 8 cm from the anus, using a polyethylenic tube. In the non-treated (Naive) group, 50% ethanol (EtOH) was injected. 15 days after the colitis induction, the animals were killed by dimethyl ether overanesthesia, and the distal colon was taken out. The stool condition and the intestinal tissue condition were evaluated by clinical indices and were scored. After its length and weight were measured, the colon tissue was frozen and stored, and analyzed for myeloperoxidase (MPO) activity measurement. A test compound was orally administered 24 hours and 1 hour before the TNBS treatment, and was orally administered continuously once a day until the day before dissection. In the control (Control) group, a solvent, 0.5% methyl cellulose solution, was administered in the same manner. As a comparative compound, a typical inflammatory bowel disease-treating agent, sulfasalazine (SSZ), 100 mg/kg was orally administered in the same manner.
The results are shown in Fig. 6. The Control group showed significant diarrheal symptom enhancement and also intestinal tissue weight increase and inflammatory symptom exacerbation indicated by MPO activity. On the other hand, in the group with oral administration of 0.3 mg/kg of the CRAC/SOC channel inhibitor, compound A (Comp A) of the present invention, a significant improvement effect of the disease activity index was confirmed, and diarrheal symptom relief, inhibition of intestinal tissue weight increase and inhibition of MPO activity exacerbation were observed. These results confirm that the CRAC/SOC channel inhibitor, compound A (Comp A) is effective for relieving both diarrheal symptom and inflammatory symptom. On the other hand, a typical inflammatory bowel disease-treating agent, SSZ also showed a relieving effect for the present models, but the effect was 1/500 times more weaker than that of the compound A (Comp A).

From the results in Examples 6 and 7, the CRAC/SOC channel inhibitor well relieved the diarrheal symptom and additionally the colitis symptom in both the oxazolone-induced acute colitis models and the TNBS-induced chronic colitis models. Accordingly, the CRAC/SOC channels play an important role both in diarrheal symptom and inflammatory symptom, and it was suggested that the drugs that control the channel could be a much more excellent inflammatory bowel disease-treating agent as compared with already-existing inflammatory bowel disease-treating agents. In particular, the compound A, or that is, 4,6-dimethyl-4'-[3,5-bis(trifluoromethyl)1H-pyrazol-1-yl]nicotinanilide has much more excellent inflammatory bowel disease-treating/diarrhea-treating effects as compared with already-existing inflammatory bowel disease-treating agents and diarrhea-treating agents.

### INDUSTRIAL APPLICABILITY

The preventive or the remedy of the present invention has a good antiinflammatory effect, and in particular, it directly acts on the CRAC/SOC channel in gastrointestinal epithelial cells to inhibit water secretion; and therefore, it is useful as a preventive or a remedy for inflammatory bowel diseases and/or diarrhea, especially for prevention or treatment of inflammatory bowel disease that are bowel diseases having, as the cardinal sign thereof, a diarrheal symptom with abdominal pain. The diarrhea includes infectious diarrhea to be induced by infection with viruses, bacteria or parasites; non-infectious diarrhea to be caused by stress, antibiotics, lactose intolerance or allergic reaction; diarrheal symptoms associated with inflammatory bowel diseases as well as diarrhea in irritable bowel syndrome.

## Claims

1. A preventive or a remedy for diarrhea, comprising, as an active ingredient thereof, a compound that inhibits a calcium release-activated calcium channel.

2. The preventive or remedy for diarrhea according to claim 1, which is a preventive or a remedy for diarrheal symptoms associated with inflammatory bowel diseases.

3. The preventive or remedy for diarrhea according to claim 1, which is a preventive or a remedy for irritable bowel syndrome.

4. The preventive or remedy for diarrhea according to any of claims 1 to 4, wherein the compound that inhibits a calcium release-activated calcium channel is a pyrazole derivative represented by the following general formula (I) or a pharmaceutically acceptable salt thereof: 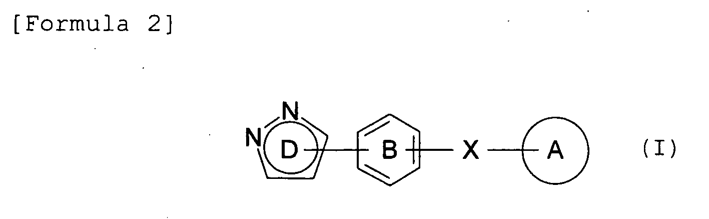 (wherein the symbols in the formula have the following meanings:
D: a 1H-pyrazol-5-yl or 1H-pyrazol-1-yl group substituted with one trifluoromethyl group and optionally having one or two substituents selected from the group consisting of lower alkyl, halogeno-lower alkyl, -CO₂H and -CO₂-lower alkyl;
X: -NHCO- or -CONH-;
A: a phenyl group optionally substituted with halogen atom(s), or a monocyclic heteroaryl group selected from thiazolyl, thiadiazolyl, thienyl and pyridyl groups, which may optionally be substituted with lower alkyl group(s);
B: a phenylene group).

5. The preventive or remedy for diarrhea according to claim 4, wherein the compound that inhibits a calcium release-activated calcium channel-inhibiting is 4,6-dimethyl-4'-[3,5-bis(trifluoromethyl)-1H-pyrazol-1-yl]nicotinanilide or a salt thereof.

6. A preventive or a remedy for inflammatory bowel diseases, comprising, as an active ingredient thereof, 4,6-dimethyl-4'-[3,5-bis(trifluoromethyl)-1H-pyrazol-1-yl]nicotinanilide or a salt thereof.

7. The preventive or remedy according to claim 6, wherein the inflammatory bowel diseases are inflammatory bowel diseases accompanied by diarrheal symptom.
